# EUROPEAN PATENT SPECIFICATION

(11) **EP 1 150 717 B1**
(45) Date of publication and mention of the grant of the patent: **22.06.2005**
(21) Application number: 00906245.6
(22) Date of filing: 29.01.2000
(51) Int. Cl.: A61K 47/48, A61K 38/09, A61K 9/10, A61K 9/14, A61K 9/19

(54) **SUSTAINED RELEASE SALTS OF PHARMACEUTICALLY ACTIVE PEPTIDES AND THEIR PRODUCTION**
SALZE VON PHARMAKOLOGISCH AKTIVEN PEPTIDEN MIT VERZÖGERTER WIRKSTOFFFREIGABE UND DEREN HERSTELLUNG
SELS D'AGENTS PHARMACEUTIQUES A LIBERATION PROLONGEE ET LEUR PRODUCTION

(30) Priority: 08.02.1999 US 119076 P
(43) Date of publication of application: 07.11.2001
(73) Proprietor: Zentaris GmbH, 60314 Frankfurt/Main (DE)
(72) Inventor: BAUER, Horst, D-91217 Hersbruck (DE); DEGER, Wolfgang, D-63755 Alzenau (DE); SARLIKIOTIS, Werner, GR-190 02 Peania (GR); DAMM, Michael, D-63322 Rödermark (DE)
(86) International application number: PCT/EP2000/000697
(87) International publication number: WO 2000/047234

(56) References cited:
- WO-A-93/24150
- WO-A-98/25642
- WO-A-98/32423
- WO-A-98/42381
- US-A- 4 581 169
- US-A- 5 773 032

## Description

### Background of the Invention

### Field of Invention

This invention relates to pharmaceutical compositions of pharmacologically-active polypeptides, which provide sustained release of the polypeptide over an extended period of time.

### Description of the Prior Art

According to the prior art (WO 98/25642) pharmaceutical formulations are claimed comprising a stable water-insoluble complex composed of a peptidic compound (e.g., a peptide, polypeptide, protein, peptidomimetic and the like), preferably a pharmaceutically active peptidic compound, and a carrier macromolecule that allow for sustained delivery of the peptidic compound in vivo upon administration of the complex. The complex according to the prior art can permit continuous delivery of a pharmaceutically active peptidic compound to a subject for prolonged periods of time, e.g., one month. Moreover, the association of the peptidic compound and the carrier macromolecule in a tight, stable complex allows for loading of high concentrations of the peptidic compound into the formulation.

The complex of the invention according to the prior art is formed by combining the peptidic compound and the carrier macromolecule under conditions such that a substantially water-insoluble complex is formed, e.g, aqueous solutions of the peptidic compound and carrier macromolecule are mixed until the complex precipitates.

The complex may be in the form of a solid (e.g., a paste, granules, a powder or a lyophilizate) or the powdered form of the complex can be pulverized finely enough to form stable liquid suspensions or semi-solid dispersions.

In a preferred embodiment, the peptidic compound of the water-insoluble complex is an LHRH analogue, more preferably an LHRH antagonist, and the carrier macromolecule is an anionic polymer, preferably sodium carboxymethylcellulose. The complex of the invention is suitable for sterilization, such as by gamma irradiation or electron beam irradiation, prior to administration in vivo.
Methods for treating a subject for a condition treatable with an LHRH analogue by administering to the subject an LHRH-analogue-containing composition of the invention are also provided.

### Problems presented by the Prior Art

For manufacturing the claimed complexes rather highly concentrated solutions (5 - 25 mg/ml) of the peptidic compound in water have to be prepared. Because of the inherent tendency of many peptidic compounds to aggregate, it can not be ensured that aggregate-free solutions in pure water can be prepared using the claimed manufacturing procedure. Depending on the water solubility of a specific peptidic compound and on the technique used to prepare this solution, the concentrated peptide solution in water may be aggregate-free or contaminated with varying concentrations and different types of peptidic aggregates and precipitates. As this highly concentrated peptidic solution is the starting material for the production of the claimed complexes, the dissolution of the peptidic compound in water is obviously a critical step.

By adding an aqueous solution of sodium carboxymethylcellulose to this not well defined and characterized, highly concentrated petide solutions in varying ratios (0,1:1 to 0,5: 1 w/w) complexes or precipitates are formed spontaneously in a non-defined, uncontrolled manner. The precipitates are collected by filtration or centrifugation, washed by rinsing with water and dried. The solid material is then powdered using a mortar and pestle. Afterwards the content of the peptidic compound is analytically determined. Due to the manufacturing procedure, the formation of stoichiometric complexes in a reproducible and well defined manner can not be guaranteed.

Additionally, by adding a solution of sodium carboxymethylcellulose (containing 6,5-9,5 % sodium according to USP) a significant amount of metall ions, i.e. sodium ions, comes into contact with the peptidic compound. Peptides and proteins might be precipitated in the presence of salts. Therefore, it is not clear, whether the complexes or precipitates described in the prior art are formed because of interactions between the peptidic compound and the functional groups of carboxymethylcellulose itsself or solely by the peptide precipitating effect of the sodium ions or by unknown and non-controllable mixtures of these two processes.

After drying and milling the peptide formulations described in the prior art are suspended in saline, which also can lead to further undesirable, uncontrolled interaction processes.

The prior art document WO 93/24150 describes salts composed of a cation derived from a peptide with at least one basic group and an anion derived from a carboxy-terminated polyester.
The salts relate exclusively to polyesters containing a terminal carboxyl group, particularly to the polymers of lactic acid and copolymers of lactic and glycolic acid. The complexes are prepared using an anion of a polyester. The polyester may have only one negative charge per polymer and accordingly it is only possible to prepare complexes where there must be at least one polymer molecule per peptide molecule.

Such polyesters liberate the peptide *in vivo* by hydrolysis of the ester bond.

A further document US 4,581,169 describes synthetic nona- and deca-peptide LHRH antagonist analogues having a novel guanido-substituted, amidine, tertiary or quaternary aminoacyl residue at position 6. The analogues may be administered in the form of slow release salts which have a low degree of solubility after administration.

US 4,581,169, however, does not disclose any example of a process for preparing such depot formulations and says nothing about the purity of these formulations.

Furthermore, it can be assumed that, since no new process is disclosed, a depot formulation of this kind has been produced by a process from the prior art in the year 1983, which did not allow the preparation of a product free from counterions.

Document WO 98/32423 describes sustained-release microspheres comprising an about 0.01 to about 10 µm particle size of a pamoic salt of physiologically active peptide and a biodegradable polymer, such as glycolic acid, lactic acid, hydroxyl butyric acid, malic acid, citric acid and polyamino acids, furthermore their complexes and a method of producing of sustained-release microspheres.

This document concems a new preparation process for so-called microspheres, which involves first forming a pamoate salt of a peptide, which is then embedded into the polymer, or by forming a three-component system.

### Summary of the Invention

The present invention provides a method of preparation of a stable, well defined, stoichiometric salt composed of an acidic or basic peptidic compound (like peptide, polypeptide protein, peptidominetic etc.) and of an ionic, basic or acidic, carrier macromolecule, respectively, allowing sustained delivery of the peptidic compound after in vivo administration of the salt of a specific peptidic compound.

According to the invention, the free base or the free acid of the peptidic compound is prepared by removing the counter ion using ion exchangers. Also, the free base or the free acid of the carrier macromolecule is prepared by removing the counter ion using ion exchangers. Thereupon, equivalent amounts of the freshly prepared peptide base or peptide acid solution, respectively, and of the counterionic-free macromolecule carrier solution are combined. The ratio of peptidic compound to carrier macromolecule (w/w) can be, for example, 1:0.1, 1:0.213, 1:0.5, 1:2.13. Non-limiting examples of conditions and procedures for preparing a water-insoluble complex of the invention are described in Examples 1 to 4.

This process results in well defined, stoichiometric and pure salts of the peptidic compound with a counterionic macromolecule. These pure salts are not contaminated by other ions, neither anions (e.g. acetate) nor cations (e.g. sodium).

Moreover, pharmaceutical compositions comprising such salts are described.

The invention particularly refers to pharmaceutical composition comprising a water-insoluble salt of a pharmaceutically active cationic peptidic compound and a counterionic carrier macromolecule obtained by the method described above, characterized in that the counterionic carrier macromolecule is carboxymethylcellulose and that the obtained composition is essentially free of other ions.

The ionic carrier macromolecule may be an anionic polymer, for example an anionic polyalcohol, a derivative or a fragment thereof.

Furthermore the ionic carrier macromolecule can be an anionic polysaccharide, a derivative or a fragment thereof. Preferably the carrier macromolecule is carboxymethylcellulose.The carrier macromolecule in the pharmaceutical composition can further be selected from the group consisting of algin, alginic acid, sodium alginate, anionic acetate polymers, ionic acrylic or methacrylic polymers and copolymers, pectin, tragacanth, xanthan gums, anionic carageenan derivatives, anionic polygalacturonic acid derivatives, sulfated and sulfonated polystyrene, sodium starch glycolate, and fragments or derivatives thereof.

The ionic carrier macromolecule can also be albumin, gelatin (type A or type B), and a fragment or derivative thereof.

Cationic polymers can also be poly-L-lysine and other polymers of basic amino acids.

The peptide in the compound is a pharmaceutically active peptidic compound and can be a mono-, di- or multivalent cationic or anionic polypeptide, wherein the polypeptide is 5 to 100 amino acids in length, preferabely 5 to 20 amino acids in length, more preferabely the peptide is 8 to 12 amino acids in length. More in detail the peptidic compound is an LHRH analogue and the LHRH analogue is an LHRH antagonist. The LHRH analogue is for example Cetrorelix, Teverelix (Antarelix, Deghenghi et al., Biomed & Pharmacother 1993, 47, 107), Abarelix (Molineaux et al., Molecular Urology 1998, 2, 265), Ganirelix (RS-26306, Nestor et al., J. Med. Chem. 1992, 35, 3942), Azaline B, Antide (ORF-23541), A-75998 (Cannon et al., J. Pharm. Sci. 1995, 84, 953), Detirelix (Andreyko et al., J. Clin. Endocrinol. Metab. 1992, 74, 399), RS-68439 , Ramorelix (HOE-2013, Stoeckemann and Sandow, J. Cancer Res. Clin. Oncol. 1993, 119, 457), Nal-Glu (ORF-21234). Structures of the above mentioned LHRH analogues are provided for example in the above cited references and in following reviews: Behre et al., GnRH antagonists: an overview, Proceedings of the 2nd World Conference on Ovulation Induction, The Parthenon Publishing Group Ltd, UK; Kutscher et al., Angew. Chem. 1997, 109, 2240.

In a preferred embodiment of the present invention the water-insoluble salt consists essentially of a pharmaceutically active peptidic compound, preferably an LHRH analogue, more preferably an LHRH antagonist, and the carrier macromolecule carboxymethylcellulose, wherein the peptide-CMC-salt has a mass ratio peptide: CMC ranging from 1:0.006 to 1:40, preferably from 1:0.04 to 1:14, more preferably from 1:0.1 to 1:5, especially from 1:0.1 to 1:3.

The pharmaceutical compositions of the invention permit sustained delivery of the peptidic compound to a subject in vivo after administration of the composition to the subject. The duration and the extent of the sustained delivery can be varied depending upon the concentration of the peptidic compound and the carrier macromolecule used to form the salt.

### Example 1

A lyophilisate of cetrorelix-CMC-salt with a mass ratio cetrorelix : CMC of 1:0.1 resembling a molar ratio cetrorelix : carboxylic function of CMC of 1:0.48 was prepared as follows. 0.22 g Na-CMC (low viscosity grade carboxymethylcellulose, Hercules) was dissolved in 40 g water and 3 g ion exchanger (Amberlite®) was added. After stirring for 20 min the ion exchanger was removed by filtration using a glas fibre filter. 2.21 g cetrorelix acetate was dissolved in 23.4 g water and 74.6 g ethanol 96 % (v/v) was added. 20 g ion exchanger (Amberlite®) was added. After stirring for 20 min the ion exchanger was removed by filtration using a glas fibre filter. The filtrated cetrorelix base solution was added under continuous stirring to the sodium-free CMC-solution yielding a clear solution. After 1 hour stirring the solution was evaporated under vacuum to remove the ethanol yielding a dispersion. Finally, the dispersion was frozen and freeze-dried.

### Example 2

A lyophilisate of cetrorelix-CMC-salt with a mass ratio cetrorelix : CMC of 1:0.213 resembling a molar ratio cetrorelix : carboxylic function of CMC of 1:1 was prepared as follows. 0.426 g Na-CMC (low viscosity grade carboxymethylcellulose, Hercules) was dissolved in 40 g water and 5 g ion exchanger (Amberlite®) was added. After stirring for 25 min the ion exchanger was removed by filtration using a glas fibre filter. 2.21 g cetrorelix acetate was dissolved in 23.4 g water and 74.6 g ethanol 96 % (v/v) was added. 20 g ion exchanger (Amberlite®) was added. After stirring for 20 min the ion exchanger was removed by filtration using a glas fibre filter. The filtrated cetrorelix base solution was added under continuous stirring to the sodium-free CMC-solution yielding a clear solution. After 1 hour stirring the solution was evaporated under vacuum to remove the ethanol yielding a dispersion. Finally, the dispersion was frozen and freeze-dried.

### Example 3

A lyophilisate of cetrorelix-CMC-salt with a mass ratio cetrorelix : CMC of 1:0.5 resembling a molar ratio cetrorelix : carboxylic function of CMC of 1:2.41 was prepared as follows. 1.1 g Na-CMC (low viscosity grade carboxymethylcellulose, Hercules) was dissolved in 200 g water and 15 g ion exchanger (Amberlite®) was added. After stirring for 20 min the ion exchanger was removed by filtration using a glas fibre filter. 2.21 g cetrorelix acetate was dissolved in 23.4 g water and 74.6 g ethanol 96 % (v/v) was added. 20 g ion exchanger (Amberlite®) was added. After stirring for 20 min the ion exchanger was removed by filtration using a glas fibre filter. The filtrated cetrorelix base solution was added under continuous stirring to the sodium-free CMC-solution yielding a solution. After 1 hour stirring the solution was evaporated under vacuum to remove the ethanol yielding a dispersion. Finally, the dispersion was frozen and freeze-dried.

### Example 4

A lyophilisate of cetrorelix-CMC-salt with a mass ratio cetrorelix : CMC of 1:2.13 resembling a molar ratio cetrorelix : carboxylic function of CMC of 1:10 was prepared as follows. 4.26 g Na-CMC (low viscosity grade carboxymethylcellulose, Hercules) was dissolved in 400 g water and 50 g ion exchanger (Amberlite®) was added. After stirring for 25 min the ion exchanger was removed by filtration using a glas fibre filter. 2.21 g cetrorelix acetate was dissolved in 23.4 g water and 74.6 g ethanol 96 % (v/v) was added. 20 g ion exchanger (Amberlite®) was added. After stirring for 20 min the ion exchanger was removed by filtration using a glas fibre filter. The filtrated cetrorelix base solution was added under continuous stirring to the sodium-free CMC-solution yielding a turbid dispersion. After 1 hour stirring the dispersion was evaporated under vacuum to remove the ethanol. Finally, the dispersion was frozen and freeze-dried.

### Example 5

The solubility of sodium-free, pure CMC-salts with varying compositions peptide-base: CMC acid was determined in isotonic Ringer solution. The cetrorelix-CMC-salts were prepared according to example 1 to 4. Additionally, the in vitro release in Ringer solution of cetrorelix out of these sodium-free CMC-salts was tested over a time period of 168 hours using a flow-through-system. The amount of cetrorelix released after 168 h is expressed as percentage of the cetrorelix dose applied in this in vitro test method.

| peptide-base:CMC (w/w) | solubility in Ringer solution in µg/ml | in vitro release in Ringer solution after 168 h in % |
|---|---|---|
| 1:0,1 | 3,5 | 23 |
| 1:0,213 | 2,7 | 30 |
| 1:0,5 | 17,5 | 63 |
| 1:2,13 | 54 | 76 |

These in vitro data of the sodium-free CMC-salts according to this invention were compared with cetrorelix complexes manufactured with Na-CMC in identical mass ratios of peptide and CMC according to the prior art (WO 98/25642).

| peptide-base:Na-CMC (m/m) | solubility in Ringer solution in µg/ml | in vitro release in Ringer solution after 168 h in % |
|---|---|---|
| 1:0,1 | 2,5 | 46 |
| 1:0,253 | 1,5 | 48 |
| 1:0,5 | 2 | 45 |
| 1:2,13 | 2 | 17 |

The elimination of sodium and acetate ions in the peptide CMC-salts is leading to significant improvements in the in vitro bevaviour of such formulations, i.e. solubility and in vitro release characteristics.

In the Na-CMC complexes according to the prior art the solubility in Ringer solution is very low and can not be modified by changing the ratio of the components peptide and Na-CMC. Thus, the release kinetics of the peptidic compound out of these formulations cannot be modified.
In contrast, within the sodium-free CMC-salts of the peptidic compound prepared according to the invention there is a clear dependence between the mass ratio of the salt components and their in vitro behaviour. An increase in the percentage of sodium-free CMC acid within such formulations leads to a significant increase in the solubility of the peptidic compound in Ringer solution. Thus, the release kinetics of the peptidic compound out of these sodium-free CMC-salt formulations can be modified and controlled. Therefore, depending on the desired release kinetics for certain clinical applications, definite CMC-salt formulations with appropiate release patterns can be made available.

### Example 6

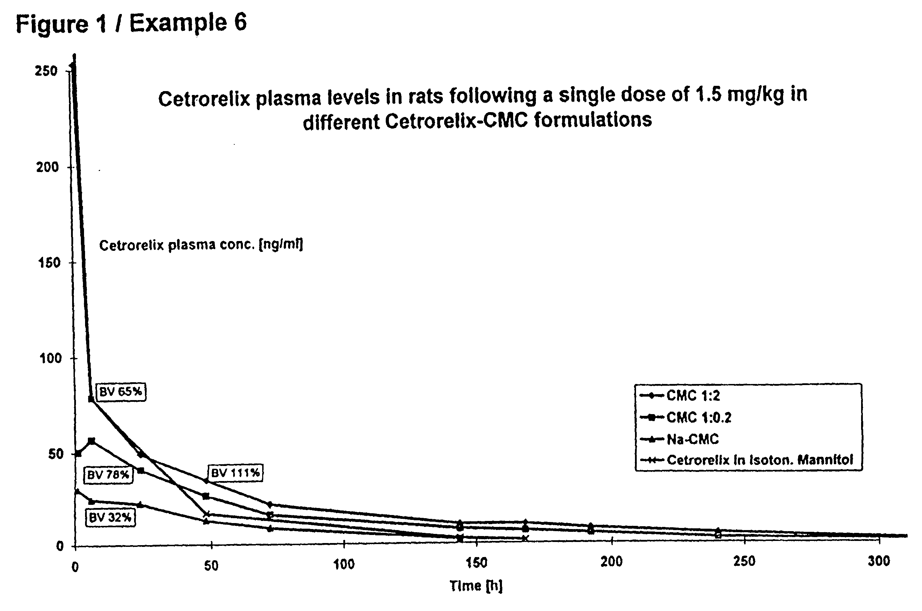

Both sodium-free CMC-salts of cetrorelix according to Examples 1 to 4 and Na-CMC-complexes of cetrorelix with equivalent mass ratios cetrorelix : CMC according to the prior art were prepared. Suspensions of such sodium-free CMC-salts of cetrorelix and of Na-CMC-complexes of cetrorelix, respectively, were prepared and a single dose was injected intramuscularly into rats in a dosage of 1,5 mg/kg. Plasma testosterone levels and plasma cetrorelix levels were determined at various time points. Additionally, at the end of the testosterone suppression the rats were killed. The muscle, into which the dose was injected, was removed and analyzed for the residual of the administered cetrorelix dose at the injection site.
Results are shown in Figure 1.
The absolute bioavailability of the Cetrorelix-CMC salts was in the range of 78%-111%. The bioavailability of the Cetrorelix-Na-CMC complexes was only 32% indicating the negative influence of the sodium ions on the properties of the formulations prepared according to the prior art.

### Example 7

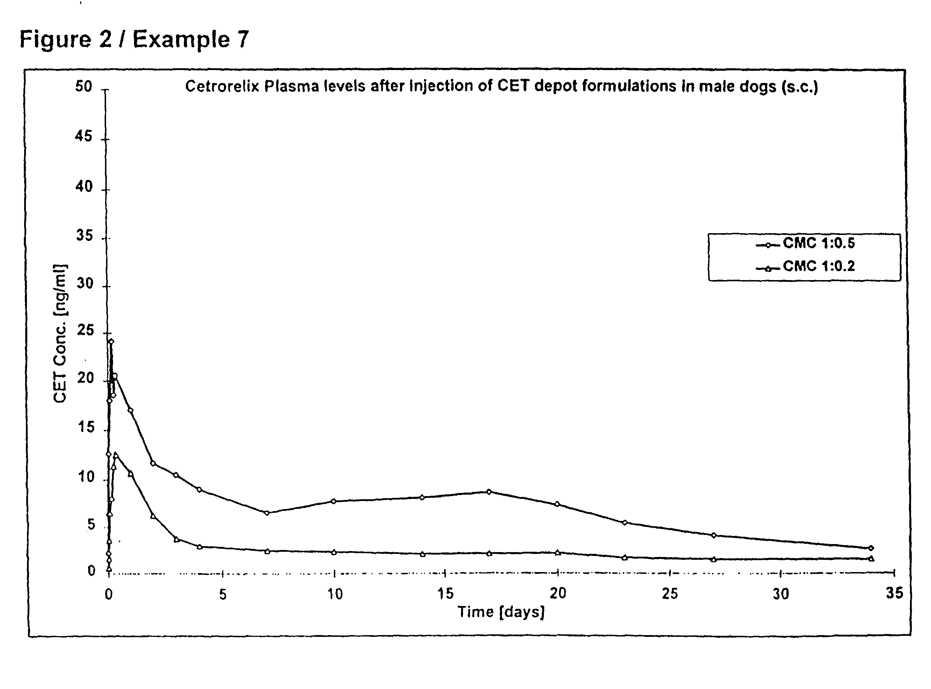

Sodium-free CMC-salts of cetrorelix according to this invention as described in previous examples were prepared as lyophitisates. The lyophilisates were dispersed in aqueous media and a single dose was injected subcutaneously into dogs in a dosage of 1,0 mg/kg. Plasma testosterone levels and plasma cetrorelix levels were determined at various time points. Results are shown in Figure 2.

## Claims

1. A method for preparing a pharmaceutical formulation, comprising a peptidic compound and a carrier macromolecule; **characterized by** the steps of forming the free ions of both compounds by removing the counter ions; combining the ionic peptidic compound and the ionic carrier macromolecule under conditions such that a water-insoluble salt of the peptidic compound and the carrier macromolecule forms; and preparing a pharmaceutical formulation comprising the water insoluble salt.

2. The method according to claim 1, wherein the counter ion is removed by using an ion exchanger.

3. The method according to claim 1 or 2, wherein a solution of the ionic peptidic compound and a solution of the carrier macromolecule are fresh prepared before combined to form a water-insoluble salt of the peptidic compound and the carrier macromolecule.

4. The method according to any one of claims 1 to 3, wherein a solution of the ionic peptidic compound and a solution of the carrier macromolecule are combined to form a water-insoluble salt of the peptidic compound and the carrier macromolecule.

5. The method according to claim 1 or 2, further comprising sterilizing the water-insoluble salt by gamma irradiation or electron beam irradiation.

6. The method according to claim 1 or 2, wherein the water-insoluble salt is formed using aseptic procedures.

7. The method according to claim 1 or 2, wherein the peptidic compound is cationic and the carrier macromolecule is anionic.

8. The method according to claim 1 or 2, wherein the peptidic compound is anionic and the carrier macromolecule is cationic.

9. The method according to claim 1 or 2, wherein the peptidic compound is a mono-, di- or multivalent cationic or anionic peptide.

10. The method according to claim 1 or 2, wherein the peptidic compound is a mono-, di- or multivalent ampholytic peptide.

11. The method according to claim 1 or 2, wherein the ionic peptidic compound is 5 to 100 amino acids in length.

12. The method according to claim 1 or 2, wherein the ionic peptidic compound is 5 to 20 amino acids in length.

13. The method according to claim 1 or 2, wherein the ionic peptidic compound is 8 to 12 amino acids in length.

14. The method according to claim 1 or 2, wherein the ionic carrier macromolecule is an anionic polymer.

15. The method according to claim 1 or 2, wherein the ionic carrier macromolecule is an ampholytic polymer.

16. The method according to claim 1 or 2, wherein the ionic carrier macromolecule is an anionic polyalcohol, a derivative or a fragment thereof.

17. The method according to claim 1 or 2, wherein the ionic carrier macromolecule is an anionic polysaccharide, a derivative or a fragment thereof, or a pharmaceutically acceptable salt thereof.

18. The method according to claim 1 or 2, wherein the ionic carrier macromolecule is carboxymethylcellulose.

19. The method according to claim 1 or 2, wherein the ionic carrier macromolecule is selected from the group consisting of algin, alginic acid, sodium alginate, anionic acetate polymers, ionic acrylic or methacrylic polymers and copolymers, pectin, tragacanth, xanthan gums, anionic carageenan derivatives, anionic polygalacturonic acid derivatives, sulfated and sulfonated polystyrene, sodium starch glycolate, and fragments or derivatives thereof, respectively.

20. The method according to claim 1 or 2, wherein the ionic carrier macromolecule is selected from the group consisting of albumins, gelatin type A, gelatin type B, and fragments or derivatives thereof.

21. The method according to claim 1 or 2, wherein the ionic carrier macromolecule is a cationic macromolecule preferably poly-L-lysine and other polymers of basic amino acids.

22. The method according to claim 1 or 2, wherein the peptidic compound is an LHRH analogue.

23. The method according to claim 22, wherein the LHRH analogue is an LHRH antagonist.

24. The method according to claim 23, wherein the LHRH antagonist is selected from the group consisting of Cetrorelix, Teverelix, Abarelix, Ganirelix RS-26306, Azaline B, Antide ORF-23541, A-75998, Detiretix, RS-68439, Ramorelix HOE-2013 or Nal-Glu ORF-21234.

25. The method according to claim 24, wherein the LHRH antagonist is Cetrorelix.

26. A pharmaceutical composition, comprising a water-insoluble salt of a pharmaceutically active cationic peptidic compound and a counterionic carrier macromolecule, whereby the water-insoluble salt is obtainable by a method according to the method of claim 1, **characterized in that** the counterionic carrier macromolecule is carboxymethylcellulose and that the obtained pharmaceutical composition is essentially free of other ions.

27. The pharmaceutical composition according to claim 26, wherein the formation of the water-insoluble salt can be mediated additionally at least in part by hydrogen bonding between the pharmaceutically active peptidic compound and the carrier macromolecule.

28. The pharmaceutical composition according to claim 26, wherein the formation of the water-insoluble salt can be mediated additionally at least in part by hydrophobic interactions between the pharmaceutically active ionic peptidic compound and the counterionic carrier macromolecule.

29. The pharmaceutical composition according to claim 26, wherein a single dose of the water-insoluble salt provides sustained delivery of the pharmaceutically active peptide to a subject for at least one week after the pharmaceutical composition is administered to the subject.

30. The pharmaceutical composition according to claim 26, wherein a single dose of the water-insoluble salt provides sustained delivery of the pharmaceutically active peptide to a subject for at least two weeks after the pharmaceutical composition is administered to the subject.

31. The pharmaceutical composition according to claim 26, wherein a single dose of the water-insoluble salt provides sustained delivery of the pharmaceutically active peptide to a subject for at least three weeks after the pharmaceutical composition is administered to the subject.

32. The pharmaceutical composition according to claim 26, wherein a single dose of the water-insoluble salt provides sustained delivery of the pharmaceutically active peptide to a subject for at least four weeks after the pharmaceutical composition is administered to the subject.

33. The pharmaceutical composition according to claim 26, wherein the pharmaceutically active ionic peptidic compound is a mono-, di- or multivalent cationic peptide.

34. The pharmaceutical composition according to claim 26, wherein the ionic peptidic compound is 5 to 100 amino acids, preferably 5 to 20 amino acids, more preferably 8 to 12 amino acids in length.

35. The pharmaceutical composition according to any one of claims 26 to 34, which is a dry solid.

36. The pharmaceutical composition according to any one of claims 26 to 34, which is a liquid suspension or semi-solid dispersion.

37. The pharmaceutical composition according to claim 26, wherein the macromolecule is CMC and the peptide-CMC-salt has a mass ratio peptide : CMC ranging from 1:0.006 to 1:40, preferably from 1:0.04 to 1:14, more preferably from 1:0.1 to 1:5, especially from 1:0.1 to 1:3.

38. The pharmaceutical composition according to claim 26 or 37, wherein the peptidic compound is an LHRH analogue.

39. The pharmaceutical composition according to claim 38, wherein the LHRH analogue is an LHRH antagonist.

40. The pharmaceutical composition according to claim 39, wherein the LHRH antagonist is selected from the group consisting of Cetrorelix, Teverelix, Abarelix, Ganirelix RS-26306, Azaline B, Antide ORF-23541, A-75998, Detirelix, RS-68439, Ramorelix HOE-2013, Nal-Glu ORF-21234.

41. The pharmaceutical composition according to claim 40, wherein the LHRH antagonist is cetrorelix.

42. The pharmaceutical composition according to claim 37 or 41, comprising a cetrorelix-CMC-complex with a mass ratio cetrorelix : CMC of 1:0.1 or 1:0.213 or 1:0.5 or of 1:2.13.

## Patentansprüche

1. Verfahren zur Herstellung einer eine peptidische Verbindung und ein Trägermakromolekül enthaltenden pharmazeutischen Formulierung, **dadurch gekennzeichnet, daß** man die freien Ionen beider Verbindungen bildet, indem man die Gegenionen entfernt, die ionische peptidische Verbindung und das ionische Trägermakromolekül unter Bedingungen vereinigt, bei denen sich ein wasserunlösliches Salz der peptidischen Verbindung und des Trägermakromoleküls bildet, und eine das wasserunlösliche Salz enthaltende pharmazeutische Formulierung herstellt.

2. Verfahren nach Anspruch 1, bei dem man das Gegenion mit einem Ionenaustauscher entfernt.

3. Verfahren nach Anspruch 1 oder 2, bei dem man eine Lösung der ionischen peptidischen Verbindung und eine Lösung des Trägermakromoleküls vor deren Vereinigung unter Bildung eines wasserunlöslichen Salzes der peptidischen Verbindung und des Trägermakromoleküls frisch zubereitet.

4. Verfahren nach einem der Ansprüche 1 bis 3, bei dem man eine Lösung der ionischen peptidischen Verbindung und eine Lösung des Trägermakromoleküls unter Bildung eines wasserunlöslichen Salzes der peptidischen Verbindung und des Trägermakromoleküls vereinigt.

5. Verfahren nach Anspruch 1 oder 2, bei dem man weiterhin das wasserunlösliche Salz durch Gammabestrahlung oder Bestrahlung mit Elektronenstrahlen sterilisiert.

6. Verfahren nach Anspruch 1 oder 2, bei dem das wasserunlösliche Salz unter Anwendung von aseptischen Vorschriften gebildet wird.

7. Verfahren nach Anspruch 1 oder 2, bei dem die peptidische Verbindung kationisch und das Trägermakromolekül anionisch ist.

8. Verfahren nach Anspruch 1 oder 2, bei dem die peptidische Verbindung anionisch und das Trägermakromolekül kationisch ist.

9. Verfahren nach Anspruch 1 oder 2, bei dem es sich bei der peptidischen Verbindung um ein mono-, di- oder multivalentes kationisches oder anionisches Peptid handelt.

10. Verfahren nach Anspruch 1 oder 2, bei dem es sich bei der peptidischen Verbindung um ein mono-, di- oder multivalentes ampholytisches Peptid handelt.

11. Verfahren nach Anspruch 1 oder 2, bei dem die ionische Peptidverbindung eine Länge von 5 bis 100 Aminosäuren aufweist.

12. Verfahren nach Anspruch 1 oder 2, bei dem die ionische Peptidverbindung eine Länge von 5 bis 20 Aminosäuren aufweist.

13. Verfahren nach Anspruch 1 oder 2, bei dem die ionische Peptidverbindung eine Länge von 8 bis 12 Aminosäuren aufweist.

14. Verfahren nach Anspruch 1 oder 2, bei dem es sich bei dem ionischen Trägermakromolekül um ein anionisches Polymer handelt.

15. Verfahren nach Anspruch 1 oder 2, bei dem es sich bei dem ionischen Trägermakromolekül um ein ampholytisches Polymer handelt.

16. Verfahren nach Anspruch 1 oder 2, bei dem es sich bei dem ionischen Trägermakromolekül um einen anionischen Polyalkohol oder ein Derivat oder ein Fragment davon handelt.

17. Verfahren nach Anspruch 1 oder 2, bei dem es sich bei dem ionischen Trägermakromolekül um ein anionisches Polysaccharid oder ein Derivat oder ein Fragment davon oder ein pharmazeutisch unbedenkliches Salz davon handelt.

18. Verfahren nach Anspruch 1 oder 2, bei dem es sich bei dem ionischen Trägermakromolekül um Carboxymethylcellulose handelt.

19. Verfahren nach Anspruch 1 oder 2, bei dem das ionische Trägermakromolekül aus der aus Algin, Alginsäure, Natriumalginat, anionischen Acetatpolymeren, ionischen acrylischen oder methacrylischen Polymeren und Copolymeren, Pectin, Tragacanth, Xanthan, anionischen Carageenanderivaten, anionischen Polygalacturonsäurederivaten, sulfatiertem und sulfoniertem Polystyrol, Natriumstärkeglycolat und Fragmenten bzw. Derivaten davon bestehenden Gruppe ausgewählt ist.

20. Verfahren nach Anspruch 1 oder 2, bei dem das ionische Trägermakromolekül aus der aus Albuminen, Gelatine Typ A, Gelatine Typ B und Fragmenten oder Derivaten davon bestehenden Gruppe ausgewählt ist.

21. Verfahren nach Anspruch 1 oder 2, bei dem es sich bei dem ionischen Trägermakromolekül um ein kationisches Makromolekül, vorzugsweise Poly-L-lysin und andere polymere basische Aminosäuren, handelt.

22. Verfahren nach Anspruch 1 oder 2, wobei es sich bei der peptidischen Verbindung um ein LHRH-Analogon handelt.

23. Verfahren nach Anspruch 22, wobei es sich bei dem LHRH-Analogon um einen LHRH-Antagonisten handelt.

24. Verfahren nach Anspruch 23, wobei der LHRH-Antagonist aus der aus Cetrorelix, Teverelix, Abarelix, Ganirelix, RS-26306, Azaline B, Antide ORF-23541, A-75998, Detirelix, RS-68439, Ramorelix HOE-2013 oder Nal-Glu ORF-21234 bestehenden Gruppe ausgewählt ist.

25. Verfahren nach Anspruch 24, bei dem es sich bei dem LHRH-Antagonisten um Cetrorelix handelt.

26. Pharmazeutische Zusammensetzung, enthaltend ein wasserunlösliches Salz einer pharmazeutisch wirksamen kationischen peptidischen Verbindung und ein gegenionisches Trägermakromolekül, wobei das wasserunlösliche Salz durch ein Verfahren gemäß dem Verfahren von Anspruch 1 erhältlich ist, **dadurch gekennzeichnet, daß** es sich bei dem gegenionischen Trägermakromolekül um Carboxymethylcellulose handelt und die erhaltene pharmazeutische Zusammensetzung im wesentlichen frei von anderen Ionen ist.

27. Pharmazeutische Zusammensetzung nach Anspruch 26, wobei die Bildung des wasserunlöslichen Salzes zusätzlich zumindest teilweise durch Wasserstoffbrückenbindung zwischen der pharmazeutisch wirksamen peptidischen Verbindung und dem Trägermakromolekül vermittelt werden kann.

28. Pharmazeutische Zusammensetzung nach Anspruch 26, wobei die Bildung des wasserunlöslichen Salzes zusätzlich zumindest teilweise durch hydrophobe Wechselwirkungen zwischen der pharmazeutisch wirksamen peptidischen Verbindung und dem gegenionischen Trägermakromolekül vermittelt werden kann.

29. Pharmazeutische Zusammensetzung nach Anspruch 26, wobei eine Einzeldosis des wasserunlöslichen Salzes einem Patienten über einen Zeitraum von wenigstens einer Woche nach der Verabreichung der pharmazeutischen Zusammensetzung an den Patienten das pharmazeutisch wirksame Peptid anhaltend zuführt.

30. Pharmazeutische Zusammensetzung nach Anspruch 26, wobei eine Einzeldosis des wasserunlöslichen Salzes einem Patienten über einen Zeitraum von wenigstens zwei Wochen nach der Verabreichung der pharmazeutischen Zusammensetzung an den Patienten das pharmazeutisch wirksame Peptid anhaltend zuführt.

31. Pharmazeutische Zusammensetzung nach Anspruch 26, wobei eine Einzeldosis des wasserunlöslichen Salzes einem Patienten über einen Zeitraum von wenigstens drei Wochen nach der Verabreichung der pharmazeutischen Zusammensetzung an den Patienten das pharmazeutisch wirksame Peptid anhaltend zuführt.

32. Pharmazeutische Zusammensetzung nach Anspruch 26, wobei eine Einzeldosis des wasserunlöslichen Salzes einem Patienten über einen Zeitraum von wenigstens vier Wochen nach der Verabreichung der pharmazeutischen Zusammensetzung an den Patienten das pharmazeutisch wirksame Peptid anhaltend zuführt.

33. Pharmazeutische Zusammensetzung nach Anspruch 26, wobei es sich bei der pharmazeutisch wirksamen ionischen peptidischen Verbindung um ein mono-, di- oder multivalentes kationisches Peptid handelt.

34. Pharmazeutische Zusammensetzung nach Anspruch 26, wobei die ionische peptidische Verbindung eine Länge von 5 bis 100 Aminosäuren, vorzugsweise 5 bis 20 Aminosäuren und besonders bevorzugt 8 bis 12 Aminosäuren aufweist.

35. Pharmazeutische Zusammensetzung nach einem der Ansprüche 26 bis 34, bei der es sich um einen trockenen Feststoff handelt.

36. Pharmazeutische Zusammensetzung nach einem der Ansprüche 26 bis 34, bei der es sich um eine flüssige Suspension oder halbfeste Dispersion handelt.

37. Pharmazeutische Zusammensetzung nach Anspruch 26, wobei es sich bei dem Makromolekül um CMC handelt und das Peptid-CMC-Salz ein Massenverhältnis von Peptid zu CMC im Bereich von 1:0,006 bis 1:40, vorzugsweise 1:0,04 bis 1:14, besonders bevorzugt 1:0,1 bis 1:5 und insbesondere 1:0,1 bis 1:3 aufweist.

38. Pharmazeutische Zusammensetzung nach Anspruch 26 oder 37, wobei es sich bei der peptidischen Verbindung um ein LHRH-Analogon handelt.

39. Pharmazeutische Zusammensetzung nach Anspruch 38, wobei es sich bei dem LHRH-Analogon um einen LHRH-Antagonisten handelt.

40. Pharmazeutische Zusammensetzung nach Anspruch 39, wobei der LHRH-Antagonist aus der Cetrorelix, Teverelix, Abarelix, Ganirelix RS-26306, Azaline B, Antide ORF-23541, A-75998, Detirelix, RS-68439, Ramorelix HOE-2013, Nal-Glu ORF-21234 bestehenden Gruppe ausgewählt ist.

41. Pharmazeutische Zusammensetzung nach Anspruch 40, wobei es sich bei dem LHRH-Antagonisten um Cetrorelix handelt.

42. Pharmazeutische Zusammensetzung nach Anspruch 37 oder 41, enthaltend einen Cetrorelix-CMC-Komplex mit einem Massenverhältnis von Cetrorelix zu CMC von 1:0,1 oder 1:0,213 oder 1:0,5 oder 1:2,13.

## Revendications

1. Procédé de préparation d'une formulation pharmaceutique, comprenant un composé peptidique et une macromolécule porteuse,
**caractérisé par**
les étapes de formation des deux composés sans ions par élimination des contre-ions ; la combinaison du composé peptidique ionique et de la macromolécule porteuse ionique dans des conditions telles qu'un sel insoluble dans l'eau du composé peptidique et de la macromolécule porteuse se forme ; et préparation d'une formulation pharmaceutique comprenant le sel insoluble dans l'eau.

2. Procédé selon la revendication 1,
**caractérisé en ce que**
le contre-ion est éliminé en utilisant un échangeur d'ions.

3. Procédé selon la revendication 1 ou 2,
**caractérisé en ce qu'**
une solution du composé peptidique ionique et une solution de la macromolécule porteuse sont fraîchement préparées avant d'être combinées pour former un sel insoluble dans l'eau du composé peptidique et de la macromolécule porteuse.

4. Procédé selon l'une quelconque des revendications 1 à 3,
**caractérisé en ce qu'**
une solution du composé peptidique ionique et une solution de la macromolécule porteuse sont combinées pour former un sel insoluble dans l'eau du composé peptidique et de la macromolécule porteuse.

5. Procédé selon la revendication 1 ou 2, comprenant en plus la stérilisation du sel insoluble dans l'eau par irradiation gamma ou par irradiation par faisceau d'électrons.

6. Procédé selon la revendication 1 ou 2,
**caractérisé en ce que**
le sel insoluble dans l'eau est formé en utilisant des procédés aseptiques.

7. Procédé selon la revendication 1 ou 2,
**caractérisé en ce que**
le composé peptidique est cationique et la macromolécule porteuse est anionique.

8. Procédé selon la revendication 1 ou 2,
**caractérisé en ce que**
le composé peptidique est anionique et la macromolécule porteuse est cationique.

9. Procédé selon la revendication 1 ou 2,
**caractérisé en ce que**
le composé peptidique est un peptide mono-, di- ou multivalent cationique ou anionique.

10. Procédé selon la revendication 1 ou 2,
**caractérisé en ce que**
le composé peptidique est un peptide mono-, di- ou multivalent ampholyte.

11. Procédé selon la revendication 1 ou 2,
**caractérisé en ce que**
le composé peptidique est constitué de 5 à 100 acides aminés.

12. Procédé selon la revendication 1 ou 2,
**caractérisé en ce que**
le composé peptidique est constitué de 5 à 20 acides aminés.

13. Procédé selon la revendication 1 ou 2,
**caractérisé en ce que**
le composé peptidique est constitué de 8 à 12 acides aminés.

14. Procédé selon la revendication 1 ou 2,
**caractérisé en ce que**
la macromolécule porteuse ionique est un polymère anionique.

15. Procédé selon la revendication 1 ou 2,
**caractérisé en ce que**
la macromolécule porteuse ionique est un polymère ampholyte.

16. Procédé selon la revendication 1 ou 2,
**caractérisé en ce que**
la macromolécule porteuse ionique est un polyol anionique, un dérivé ou un fragment de celui-ci.

17. Procédé selon la revendication 1 ou 2,
**caractérisé en ce que**
la macromolécule porteuse ionique est un polysaccharide anionique, un dérivé ou un fragment de celui-ci, ou un sel acceptable en pharmacie de celui-ci.

18. Procédé selon la revendication 1 ou 2,
**caractérisé en ce que**
la macromolécule porteuse ionique est de la carboxyméthylcellulose.

19. Procédé selon la revendication 1 ou 2,
**caractérisé en ce que**
la macromolécule porteuse ionique est choisi dans le groupe comprenant l'algine, l'acide alginique, l'alginate de sodium, les polymères d'acétate ioniques, les polymères et copolymères acryliques ou méthacryliques ioniques, la pectine, l'adragante, les gommes de xanthane, les dérivés de la carraghénine anioniques, les dérivés anioniques de l'acide polygalacturonique, le polystyrène sulfaté et sulfoné, le glycolate de sodium d'amidon, et des fragments ou des dérivés de ceux-ci.

20. Procédé selon la revendication 1 ou 2,
**caractérisé en ce que**
la macromolécule porteuse ionique est choisi dans le groupe comprenant des albumines, la gélatine type A, la gélatine type B, et des fragments ou des dérivés de celles-ci.

21. Procédé selon la revendication 1 ou 2,
**caractérisé en ce que**
la macromolécule porteuse ionique est une macromolécule cationique, de préférence la poly-L-lysine et autres polymères d'acides aminés basiques.

22. Procédé selon la revendication 1 ou 2,
**caractérisé en ce que**
le composé peptidique est un analogue LHRH.

23. Procédé selon la revendication 22,
**caractérisé en ce que**
l'analogue LHRH est un antagoniste LHRH.

24. Procédé selon la revendication 23,
**caractérisé en ce que**
l'antagoniste LHRH est choisi dans le groupe comprenant Cetrorelix, Teverelix, Abarelix, Ganirelix RS-26306, Azaline B, Antide ORF-23541, A-75998, Detirelix, RS-68439, Ramorelix HOE-2013 ou Nal-Glu ORF-21234.

25. Procédé selon la revendication 24,
**caractérisé en ce que**
l'antagoniste LHRH est Cetrorelix.

26. Composition pharmaceutique, comprenant un sel insoluble d'un composé peptidique cationique actif en pharmacie et d'une macromolécule porteuse contre-ion, à partir desquels le sel insoluble dans l'eau est obtenu par un procédé selon le procédé de la revendication 1,
**caractérisée en ce que**
la macromolécule porteuse contre-ion est la carboxyméthylcellulose, et la composition pharmaceutique obtenue est essentiellement sans autres ions.

27. Composition pharmaceutique selon la revendication 26,
**caractérisée en ce que**
la formation du sel insoluble dans l'eau peut en plus être médiatisée au moins en partie par une liaison hydrogène entre le composé peptidique ionique actif en pharmacie et la macromolécule porteuse.

28. Composition pharmaceutique selon la revendication 26,
**caractérisée en ce que**
la formation du sel insoluble dans l'eau peut en plus être médiatisée au moins en partie par des interactions hydrophobes entre le composé peptidique ionique actif en pharmacie et la macromolécule porteuse contre-ion.

29. Composition pharmaceutique selon la revendication 26,
**caractérisée en ce qu'**
une dose unique du sel insoluble dans l'eau apporte une libération prolongée du peptide actif en pharmacie à un sujet pendant au moins une semaine après que la composition pharmaceutique ait été administrée au sujet.

30. Composition pharmaceutique selon la revendication 26,
**caractérisée en ce qu'**
une dose unique du sel insoluble dans l'eau apporte une libération prolongée du peptide actif en pharmacie à un sujet pendant au moins deux semaines après que la composition pharmaceutique ait été administrée au sujet.

31. Composition pharmaceutique selon la revendication 26,
**caractérisée en ce qu'**
une dose unique du sel insoluble dans l'eau apporte une libération prolongée du peptide actif en pharmacie à un sujet pendant au moins trois semaines après que la composition pharmaceutique ait été administrée au sujet.

32. Composition pharmaceutique selon la revendication 26,
**caractérisée en ce qu'**
une dose unique du sel insoluble dans l'eau apporte une libération prolongée du peptide actif en pharmacie à un sujet pendant au moins quatre semaines après que la composition pharmaceutique ait été administrée au sujet.

33. Composition pharmaceutique selon la revendication 26,
**caractérisée en ce que**
le composé peptidique ionique actif en pharmacie est un peptide mono, di ou multivalent cationique.

34. Composition pharmaceutique selon la revendication 26,
**caractérisée en ce que**
le composé peptidique comprend de 5 à 100 acides aminés, de préférence de 5 à 20 acides aminés, plus particulièrement de 8 à 12 acides aminés.

35. Composition pharmaceutique selon la revendication 26, qui est un solide sec.

36. Composition pharmaceutique selon la revendication 26, qui est une suspension liquide ou une dispersion semi-solide.

37. Composition pharmaceutique selon la revendication 26,
**caractérisée en ce que**
la macromolécule est de la CMC et que le sel peptide-CMC a un rapport pondéral peptide/CMC allant de 1/0,006 à 1/40, de préférence de 1/0,04 à 1/14, plus particulièrement de 1/0,1 à 1/5, spécifiquement de 1/0,1 à 1/3.

38. Composition pharmaceutique selon la revendication 26 ou 37,
**caractérisée en ce que**
le composé peptidique est un analogue LHRH.

39. Composition pharmaceutique selon la revendication 38,
**caractérisée en ce que**
l'analogue LHRH est un antagoniste LHRH.

40. Composition pharmaceutique selon la revendication 39,
**caractérisée en ce que**
l'antagoniste LHRH est choisi dans le groupe comprenant Cetrorelix, Teverelix, Abarelix, Ganirelix RS-26306, Azaline B, Antide ORF-23541, A-75998, Detirelix, RS-68439, Ramorelix HOE-2013 ou Nal-Glu ORF-21234.

41. Composition pharmaceutique selon la revendication 40,
**caractérisée en ce que**
l'antagoniste LHRH est Cetrorelix.

42. Composition pharmaceutique selon la revendication 37 ou 41, comprenant un complexe cetrorelix-CMC avec un rapport pondéral cetrorelix/CMC de 1/0,1 ou de 1/0,213 ou de 1/0,5 ou de 1/2,13.
